(19) Europäisches Patentamt
European Patent Office
Office européen des brevets

(11) Veröffentlichungsnummer: **0 400 283 B1**

(12) **EUROPÄISCHE PATENTSCHRIFT**

(45) Veröffentlichungstag der Patentschrift: **11.01.95**

(51) Int. Cl.[6]: **C08F 283/06**, A61L 15/00

(21) Anmeldenummer: **90104966.8**

(22) Anmeldetag: **16.03.90**

# (54) Hydrophile quellfähige Pfropfpolymerisate, deren Herstellung und Verwendung.

(30) Priorität: **07.04.89 DE 3911433**

(43) Veröffentlichungstag der Anmeldung:
**05.12.90 Patentblatt 90/49**

(45) Bekanntmachung des Hinweises auf die Patenterteilung:
**11.01.95 Patentblatt 95/02**

(84) Benannte Vertragsstaaten:
**BE CH DE DK ES FR GB IT LI NL SE**

(56) Entgegenhaltungen:
**EP-A- 0 003 235**
**EP-A- 0 316 792**
**WO-A-87/05611**
**DE-A- 3 401 813**

(73) Patentinhaber: **CASSELLA Aktiengesellschaft**
**Hanauer Landstrasse 526**
**D-60386 Frankfurt (DE)**

(72) Erfinder: **Engelhardt, Friedrich, Dr.**
**Hünfelder Strasse 20**
**D-6000 Frankfurt am Main 61 (DE)**
Erfinder: **Riegel, Ulrich**
**Steinäckerstrasse 6**
**D-6000 Frankfurt am Main 61 (DE)**

(74) Vertreter: **Muley, Ralf, Dr.**
**Cassella AG,**
**Patentabteilung,**
**Hanauer Landstrasse 526**
**D-60386 Frankfurt (DE)**

**EP 0 400 283 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft hydrophile, quellbare Pfropfpolymerisate, die in statistischer Verteilung zu 0,5 bis 20 Gew.% aus Resten der allgemeinen Formel I

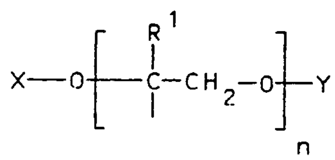

(I)

zu 79 bis 99 Gew.% aus eine saure Gruppe enthaltenden Resten der allgemeinen Formel II

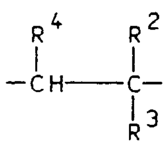

(II)

und zu 0,1 bis 2 Gew.% aus Resten eines Vernetzers, die aus Monomeren mit mindestens zwei olefinisch ungesättigten Doppelbindungen hervorgegangen sind, bestehen, wobei
X ($C_1$-$C_{22}$)-Alkyl, Aryl, Aralkyl oder Y,
Y $COCH_3$, $CH_2COOR^2$, $COCH_2CH_2COOH$,

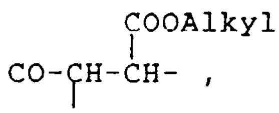

$COOR^2$, $CH_2COCH_2COOR^2$,

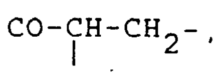

$SO_3H$ oder

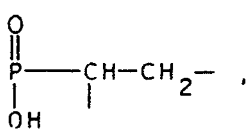

n 2 bis 300,
$R^1$ Wasserstoff oder Methyl,
$R^2$ unabhängig voneinander Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

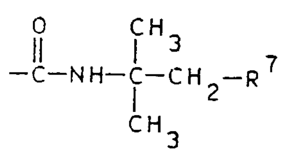

worin $R^7$ für die Sulfonylgruppe oder die Phosphonylgruppe steht,
$R^4$ Wasserstoff, Methyl, Ethyl oder die Carboxylgruppe bedeuten,
sowie ihre Herstellung und Verwendung als Absorptionsmittel für Wasser und wäßrige Lösungen, zum Beispiel in Hygieneartikeln, zur Bodenverbesserung oder als Filtrationshilfsmittel.

Quellbare Polymere, die wäßrige Lösungen absorbieren, werden für die Herstellung van Tampons, Windeln, Damenbinden und anderen Hygieneartikeln sowie als wasserzurückhaltende Mittel im landwirtschaftlichen Gartenbau verwendet.

Zu bekannten Absorptionsharzen dieses Typs gehören vernetzte Carboxymethylcellulose, teilweise vernetztes Polyalkylenoxid, Hydrolysate von Stärke-Acrylnitril-Pfropfcopolymeren oder teilweise vernetzte Polyacrylsäuresalze.

Diese bekannten Polymerisate zeigen durchweg Nachteile, insbesondere bei der Absorption wäßriger Elektrolytlösung sowie Blut und Urin.

Bei hohem Absorptionsvermögen werden nach dem derzeitigen Stand der Technik zu geringe Gelfestigkeiten der gequollenen Polymerpartikel erreicht. Es bilden sich klebrige Massen, welche die Saugfähigkeit der damit hergestellten Produkte verschlechtern.

Es ist bekannt, daß durch Erhöhung der Vernetzungsdichte die Gelfestigkeit sowie die Geschwindigkeit der Flüssigkeitsaufnahme erhöht werden kann, dadurch jedoch gleichzeitig die Absorptionskapazität herabgesetzt wird. Diese Vorgehensweise ist insofern unerwünscht als die Absorptionskapazität die wichtigste Eigenschaft des Polymeren ist.

Aufgabe der vorliegenden Erfindung ist es, modifizierte Polymere, die wäßrige Lösungen absorbieren, bereitzustellen, welche eine hohe Absorptionsrate aufweisen und dabei im gequollenen Zustand nicht klebende Hydrogelpartikel hoher Gelfestigkeit bilden.

Überraschenderweise wurde nun gefunden, daß das gewünschte Eigenschaftsprofil durch die erfindungsgemäßen Pfropfpolymerisate erreicht wird, da deren makromolekulares Netzwerk physikalisch eine Erhöhung der Gelfestigkeit oder Gelstärke des gequollenen Polymeren sowie eine verbesserte Elektrolyttoleranz bewirkt.

Bevorzugte erfindungsgemäße Produkte bestehen zu 0,5 bis 15 Gew.% aus Resten der allgemeinen Formel I, 84 bis 99 Gew.% aus Resten der allgemeinen Formel II und 0,1 bis 1,8 Gew.% aus vernetzenden Strukturen, die aus Monomeren mit mindestens zwei olefinisch ungesättigten Doppelbindungen hervorgegangen sind.

Besonders bevorzugte erfindungsgemäße Produkte bestehen zu 1 bis 10,5 Gew.% aus Resten der allgemeinen Formel I, 88 bis 98,5 Gew.% aus Resten der allgemeinen Formel II und 0,3 bis 1,5 Gew.% aus vernetzenden Strukturen, die aus Monomeren mit mindestens zwei olefinisch ungesättigten Doppelbindungen hervorgegangen sind.

In den erfindungsgemäßen Pfropfcopolymerisaten können die Reste der allgemeinen Formel I alle exakt die gleiche Struktur haben, sie können sich jedoch auch hinsichtlich des Restes $R^1$ und/oder der Zahl n voneinander unterscheiden. So können sich bezüglich $R^1$ Wasserstoff und Methyl in statistischer Weise abwechseln, es können aber auch größere Polymerabschnitte aufeinander folgen, in denen $R^1$ jeweils nur Wasserstoff oder nur Methyl bedeutet.

Für X stehendes Aryl hat bevorzugt 3 bis 8 Kohlenstoffatome und bedeutet besonders bevorzugt Phenyl, tert.-Butyl-phenyl und Nonylphenyl. Für X stehendes Aralkyl hat bevorzugt 3 bis 8 Kohlenstoffatome im Aryl-und 1 bis 22 Kohlenstoffatome im Alkylrest.

Die Alkylgruppe des für Y stehenden Restes

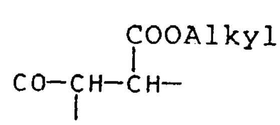

hat bevorzugt 1 bis 22 Kohlenstoffatome.
Y bedeutet bevorzugt $COCH_3$, $CH_2COOR^2$, $COCH_2CH_2COOH$, $COOR^2$, $CH_2COCH_2COOR_2$ und

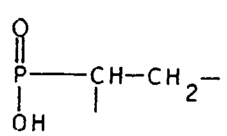
,

In den Resten der allgemeinen Formel II bedeutet $R^2$ bevorzugt Wasserstoff oder Methyl. $R^3$ steht bevorzugt für die Carboxylgruppe, die Sulfonylgruppe oder die Phosphonylgruppe. Besonders bevorzugt ist die Carboxylgruppe. $R^4$ bedeutet bevorzugt Wasserstoff.

Die genannten vernetzenden Strukturen können sich von allen geeigneten Monomeren mit mindestens zwei olefinisch ungesättigten Doppelbindungen ableiten.

Geeignete Monomere sind beispielsweise Verbindungen, die mindestens zwei Alkenylgruppen, zum Beispiel Vinyl oder Allyl, oder mindestens zwei Alkenoylgruppen, zum Beispiel Acrylat oder Methacrylat, enthalten.

Bevorzugt leiten sich die vernetzenden Strukturen von Monomeren ab, die 2, 3 oder 4 ethylenisch ungesättigte Doppelbindungen enthalten.

Besonders bevorzugt leiten sich die vernetzenden Strukturen von Trimethylolpropantriacrylat, Tetraallyloxyethan oder Methylenbisacrylamid ab.

Weitere vernetzende Strukturen können erhalten werden durch Zusatz van mehrfunktionellen Epoxiden, wie z.B. Ethylenglykoldiglycidylether oder cycloaliphatischem Diepoxid.

Ganz besonders bevorzugte erfindungsgemäße Pfropfpolymerisate sind solche, in denen mehrere der oben genannten bevorzugten oder besonders bevorzugten Merkmale enthalten sind.

Die erfindungsgemäßen Pfropfpolymerisate können durch bekannte Polymerisationsverfahren hergestellt werden. Bevorzugt ist die Polymerisation in wäßriger Lösung nach dem Verfahren der sogenannten Gelpolymerisation. Dabei werden 15-50%ige wäßrige Lösungen der Comonomeren mit bekannten geeigneten Katalysatorsystemen ohne mechanische Durchmischung unter Ausnutzung des Trommsdorff-Norrish-Effektes (Bios Final Rep. 363.22; Makromol. Chem. 1, 169 (1947)) polymerisiert.

Die Polymerisationsreaktion kann im Temperaturbereich zwischen 0°C und 130°C, vorzugsweise zwischen 10°C und 100°C, sowohl bei Normaldruck als auch unter erhöhtem Druck durchgeführt werden. Wie üblich kann die Polymerisation auch in einer Schutzgasatmosphäre, vorzugsweise unter Stickstoff, ausgeführt werden.

Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert. Butylhydroperoxid, Methyl-ethyl-keton-peroxid, Cumol-hydroperoxid, Azoverbindungen wie Azo-di-iso-butyro-nitril sowie anorganische Peroxiverbindungen wie $(NH_4)_2S_2O_8$ oder $K_2S_2O_8$ oder $H_2O_2$ gegebenenfalls in Kombination mit Reduktionsmitteln wie Natriumhydrogensulfit, und Eisen-II-Sulfat oder Redoxsysteme, welche als reduzierende Komponente eine aliphatische und aromatische Sulfinsäure, wie Benzolsulfinsäure und Toluolsulfinsäure oder Derivate dieser Säuren, enthalten, wie z.B. Mannichaddukte aus Sulfinsäure, Aldehyden und Aminoverbindungen, wie sie in der deutschen Patentschrift 1301566 beschrieben sind. Pro 100 g Gesamtmonomeren werden in der Regel 0,03 bis 2 g des Polymerisationsinitiators eingesetzt.

Durch mehrstündiges Nachheizen der Polymerisatgele im Temperaturbereich von 50-130°C, vorzugsweise 70-100°C, können die Qualitätseigenschaften der Polymerisate noch verbessert werden.

Die auf diesem Wege hergestellten, in Form wäßriger Gallerten vorliegenden erfindungsgemäßen Copolymerisate können nach mechanischer Zerkleinerung mit geeigneten Apparaten durch bekannte Trocknungsverfahren in fester Form erhalten werden und zum Einsatz gelangen.

Zweckmäßigerweise werden somit erfindungsgemäße Pfropfpolymerisate erhalten, wenn 0,5 bis 20 Gew.%, vorzugsweise 0,5 bis 15, insbesondere 1 bis 10,5 Gew.%, einer Polyalkylenoxidverbindung der allgemeinen Formel Ia

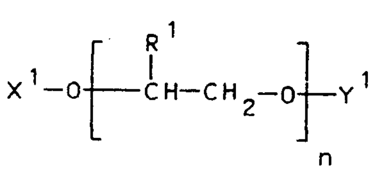

(Ia)

oder gegebenenfalls ein Alkali-, Ammonium- oder Aminsalz davon,
79 bis 99 Gew.%, vorzugsweise 84 bis 99, insbesondere 88 bis 98,5 Gew.%, einer ungesättigten Säure der allgemeinen Formel IIa

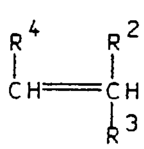

(IIa)

oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei

$X^1$ ($C_1$-$C_{22}$)-Alkyl, Aryl, Aralkyl oder Y,

$Y^1$ $COCH_3$, $CH_2COOR^2$, $COCH_2CH_2COOH$, CO-CH=CH-COOAlkyl, $COOR^2$, $CH_2COCH_2COOR^2$, CO-CH=$CH_2$, $SO_3H$ oder

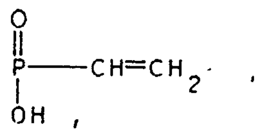

und die Reste $R^1$ bis $R^4$ und die Zahl n die oben genannten Bedeutungen haben, unter den Bedingungen der Gelpolymerisation umgesetzt werden.

Die Polyalkylenoxidverbindungen der allgemeinen Formel Ia können durch bekannte Umsetzungsreaktionen von Verbindungen mit reaktiven Gruppen, wie Anhydride, Säurechloride, Halogencarbonsäuren oder deren Estern oder Halogensulfonsäuren und Polyalkylenoxiden erhalten werden.

Bevorzugte Polyalkylenoxide sind Polypropylen- und Polyethylenoxide, Co- oder Blockcopolymere aus Ethylenoxid und Propylenoxid, Oxethylate, Oxpropylate oder Oxethyloxpropylate von aliphatischen $C_1$ bis $C_{22}$ Alkylalkoholen, Phenol, tert.Butylphenol oder Nonylphenol.

Bevorzugte Reagenzien zum Endverschluß der Polymerkette sind Chloracetessigsäure und deren Ester, Chlorameisensäure und deren Ester, Vinylphosphonsäuremono- und Dichlorid, Bernsteinsäureanhydrid, Essigsäureanhydrid, Monochloressigsäure.

Die Monomeren der Formel IIa sind bekannte Verbindungen, wie zum Beispiel Acrylsäure, Methacrylsäure, Vinylsulfonsäure, Maleinsäure, Fumarsäure, Crotonsäure, 2-Acrylamido-2-methyl-propansulfonsäure, 2-Acrylamido-2-methylpropanphosphonsäure und Vinylphosphonsäure sowie deren Halbester.

Die als Vernetzer eingesetzten polyolefinischen Monomere sind gängige Produkte. Beispiele sind Bisacrylamidoessigsäure, Trimethylolpropantriacrylat, Tetraallyloxyethan, Methylenbisacrylamid.

Die erfindungsgemäßen Pfropfpolymerisate eignen sich hervorragend als Absorptionsmittel für Wasser und wäßrige Lösungen, so daß sie vorteilhaft als wasserzurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Die folgenden Beispiele 1 bis 13 veranschaulichen die Herstellung erfindungsgemäßer Pfropfpolymerisate.

Beispiel 1:

In einen durch geschäumtes Kunststoffmaterial gut isolierten Polyethyleneimer mit einem Fassungsvermögen von 10 l werden 4920 g entsalztes Wasser vorgelegt, 1493 g Natriumbicarbonat darin dispergiert und langsam 1910 g Acrylsäure so zudosiert, daß ein Überschäumen der Reaktionslösung vermieden wird, wobei sich diese auf eine Temperatur von ca. 12 - 10°C abkühlt. Es werden nun 40 g des als Pfropfgrundlage dienenden Umsetzungsproduktes gemäß Beispiel a (siehe unten), 20 g Trimethylolpropantriacrylat, gelöst in 20 g eines Polyglykolethers auf Basis eines synthetischen $C_{12}$-$C_{15}$-Oxoalkohols mit 13 Ethylenoxid-Einheiten, 10 g eines Natrium-Diisooctylsulfosuccinates (REWOPOL V 2133 der Firma REWO, Steinau) und 30 g eines cycloaliphatischen Epoxids (DIEPOXID der Firma DEGUSSA AG) zugegeben. Bei einer Temperatur von 10 - 12°Cwerden die Initiatoren, ein Redoxsystem, bestehend aus 2,2 g 2,2'-Azobisamidinopropan-Dihydrochlorid, gelöst in 20 g Wasser, 4,4 g Kaliumperoxidisulfat, gelöst in 170 g Wasser, und 6 g Natriumpyrosulfit, gelöst in 120 g Wasser, nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85°C ansteigt, ein festes Gel entsteht. Dieses wird anschließend mechanisch zerkleinert, bei Temperaturen über 80°C getrocknet und gemahlen.

Das vorstehend beschriebene Produkt wurde in herkömmlicher Weise in eine Babywindel eingearbeitet und zeichnete sich hier durch eine besonders gute Flüssigkeitsretention aus.

Herstellung der Pfropfgrundlagen:

Beispiel a:

In 312 g eines Blockcopolymeren aus 1,03 mol Propylenoxid und 0,91 mol Ethylenoxid mit der OH-Zahl 36, werden unter Rühren bei Raumtemperatur 20,0 g Bernsteinsäureanhydrid eingetragen und diese Mischung wird unter Rühren auf 80°C erhitzt. Das Bernsteinsäureanhydrid löst sich dabei unter schwach exothermer Reaktion auf, es entsteht eine klare farblose Lösung.

Beispiel b:

In einem Vierhalskolben mit Azeotropaufsatz und Stickstoffeinleitung werden 1010 g (0,495 mol) Polypropylenglykol 2020 in 500 ml Toluol gelöst und entwässert, d.h. es wird 3 h azeotrop destilliert, wobei 43 g Wasser abgeschieden werden. Bei 105 - 110°C werden im Laufe von 30 min 112,5 g (1,1 mol) Essigsäureanhydrid zugetropft und es wird 2 h bei 105 - 110°C nachgerührt. Unter Wasserstrahlvakuum werden Toluol, Essigsäure und überschüssiges Essigsäureanhydrid abdestilliert. Als Rückstand verbleiben 1020 g eines farblosen Öls.

Beispiel c:

4621 g (1.0 mol) eines Polyglykolethers auf Basis Nonylphenol mit 100 Ethylenoxideinheiten werden geschmolzen. Bei 90 -100°C werden 102 g (1,0 mol) Acetanhydrid zugetropft, es wird 30 min gerührt und dann unter Wasserstrahlvakuum die entstandene Essigsäure abdestilliert. Es entsteht eine farblose Lösung, die bei Raumtemperatur zu einem festen Wachs erstarrt.

Beispiel d:

In einem Reaktionskolben werden 345 g eines Blockpolymeren aus 1,6 mol Propylenoxid und 0,2 mol Ethylenoxid mit der OH-Zahl 65 in 350 ml Essigsäureethylester gelöst, 40,5 g Triethylamin zugesetzt und langsam 37,8 g Monochloressigsäure zugegeben. Es wird 1 h nachgerührt, das Triethylaminhydrochlorid abgesaugt und das Lösungsmittel unter Wasserstrahlvakuum abdestilliert. Als Rückstand verbleiben 368 g eines farblosen Öls.

Beispiel e:

Umsetzung analog 1a) mit einem Copolymeren aus 0,35 mol Propylenoxid und 1,82 mol Ethylenoxid mit der OH-Zahl 17.

Beispiel f:

Umsetzung analog 1a) mit einem Copolymeren aus 1,6 mol Propylenoxid und 0,2 mol Ethylenoxid mit der OH-Zahl 65.

Beispiel g:

Umsetzung analog 1d) mit Nonylphenoloxethylat mit 30 Ethyleneinheiten und Chloracetessigsäureethylester.

Beispiel h:

Umsetzung analog 1d) mit Nonylphenoloxethylat mit 30 Ethyleneinheiten und Chlorameisensäure.

Beispiel i:

Umsetzung analog 1d) mit einem Copolymeren aus 1,03 mol Propylenoxid und 0,91 mol Ethylenoxid mit der OH-Zahl 36 und Vinylphosphonsäuremonochlorid.

Beispiel k:

Umsetzung analog 1d) mit Polypropylenglykol 2020 und Monochloressigsäure.

Beispiel l:

Umsetzung analog 1c) mit tert.Butylphenoloxethylat mit 80 Ethylenoxideinheiten und Acetanhydrid.

Beispiel m:

Umsetzung analog 1d) mit Phenoloxethylat mit 15 Propylenoxideinheiten und Vinylphosphonsäuredichlorid.

Beispiel 2:

In einem 10 Liter-Kunststoffeimer werden 4419 g Eis und 1894 g Acrylsäure vorgelegt und langsam 1573 g NaOH 50%ig zudosiert, anschließend 100 g des als Pfropfgrundlage dienenden Umsetzungsproduktes gemäß Beispiel 1a, 6 g Methylenbisacrylamid dispergiert in 100 g Wasser und 10 g Rewopol V 2133 zugegeben. Die Reaktionslösung wird auf 20°C eingestellt und anschließend mit den Initiatoren, ein Redoxsystem bestehend aus 6 g Kaliumperoxidisulfat, gelöst in 170 g Wasser, und 0,15 g Ascorbinsäure, gelöst in 120 g Wasser, versetzt und ohne Rühren stehen gelassen. Das durch Polymerisation entstehende Gel wird anschließend mechanisch zerkleinert, bei Temperaturen über 80°C getrocknet und gemahlen.

Beispiel 3:

In einem 10 Liter-Polyethyleneimer werden 5130 g entsalztes Wasser, 1888 g Acrylsäure und 50 g des als Pfropfgrundlage dienenden Umsetzungsproduktes gemäß Beispiel 1c) vorgelegt. Es werden 12 g Tetraallyloxyethan und 10 g REWOPOL V 2133 eingerührt. Nach Einstellen der Reaktionslösung auf 18 - 20°C werden die Initiatoren, 6 g Kaliumperoxidisulfat in 170 g Wasser und 0,2 g Ascorbinsäure in 20 g Wasser nacheinander zugegeben und das Reaktionsgefäß wird gut isoliert ohne Rühren stehen gelassen. Nach einsetzender Reaktion steigt die Temperatur bis auf ca. 90°C an, und es entsteht ein festes Gel. Dieses wird mechanisch durch einen Extruder zerkleinert, dem kontinuierlich 1540 g NaOH 50%ig zudosiert werden, wobei teilweise Verdampfung des Wassers erfolgt. Das flockige Polymer wird anschließend bei Temperaturen über 80°C endgetrocknet und gemahlen.

Weitere Beispiele zur Herstellung erfindungsgemäßer Pfropfpolymerisate gemäß der hier beschriebenen Beispiele 1 und 2 sind in folgender Tabelle zusammengefaßt. Die Mengenangaben bedeuten Gewichts% bezogen auf Gesamtmonomeranteil.

Folgende Abkürzungen werden benutzt:

| | |
|---|---|
| AS: | Acrylsäure |
| MAS: | Methacrylsäure |
| CTS: | Crotonsäure |
| VPS: | Vinylphosphonsäure |
| VPE: | Vinylphosphonsäurehalbester |
| AMP: | 2-Acrylamido-2-methyl-propansulfonsäure |
| AMPP: | 2-Acrylamido-2-methyl-propanphosphonsäure |
| TMPTA: | Trimethylolpropantriacrylat |
| TAE: | Tetraallyloxyethan |
| MBA: | Methylenbisacrylamid |

| Beispiel | hergestellt analog Beispiel | AS (%) | MAS (%) | AMP (%) | AMPP (%) | VPS (%) | VPF (%) | CTS (%) | Pfropfgrundlage gemäß Beispiel | Pfropfgrundlage (%) | MBA (%) | TMPTA (%) | TAE (%) | Neutralisationsgrad (%) |
|---|---|---|---|---|---|---|---|---|---|---|---|---|---|---|
| 4 | 1 | 94,4 | | | | | | | 1e | 5 | | 0,6 | | 45 |
| 5 | 1 | 94,4 | | | | | | | 1f | 5 | | 0,6 | | 45 |
| 6 | 1 | 89,4 | | | | | | | 1g | 10 | | 0,6 | | 45 |
| 7 | 1 | 94,4 | | | | | | | 1h | 5 | | 0,6 | | 45 |
| 8 | 1 | 94,4 | | | | | | | 1i | 5 | | 0,6 | | 45 |
| 9 | 1 | 94,4 | | | | | | | 1k | 5 | | 0,6 | | 45 |
| 10 | 1 | 88,5 | | | | | | | 1l | 10 | | 1,5 | | 70 |
| 11 | 1 | 94,4 | | | | | | | 1m | 5 | | 0,6 | | 45 |
| 12 | 2 | 89,7 | | | | | | | 1c | 10 | 0,3 | | | 75 |
| 13 | 2 | 89,4 | | | | | | | 1c | 10 | | | 0,6 | 78 |
| 14 | 1 | 98,4 | | | | | | | 1c | 1 | | 0,6 | | 45 |
| 15 | 1 | 70,0 | 10,0 | 9,5 | | | | | 1c | 10 | | | 0,5 | 48 |
| 16 | 2 | 65,25 | | 25,0 | | 4,0 | | | 1c | 5 | 0,25 | | | 45 |
| 17 | 2 | 75,0 | 5,0 | 10,0 | | | 4,2 | | 1c | 5 | | 0,8 | | 60 |
| 18 | 2 | 85,0 | | 5,0 | 4,5 | | | | 1c | 5 | 0,5 | | | 70 |
| 19 | 1 | 72,4 | | 20,0 | | 4,2 | | | 1c | 3 | 0,4 | | | 80 |
| 20 | 1 | 81,0 | 10,0 | | | | 4,0 | | 1c | 4 | | 1,0 | | 36 |
| 21 | 2 | 90,0 | | | 4,6 | | | | 1c | 5 | | | 0,4 | 25 |
| 22 | 2 | 79,0 | | 19,0 | | | | | 1c | 1 | | | 1,0 | 40 |
| 23 | 1 | 72,0 | | 19,3 | | | | 5,0 | 1c | 3 | | | 0,7 | 48 |
| 24 | 1 | 90,0 | | | | 1,0 | | | 1c | 8 | | | 1,0 | 32 |

**Patentansprüche**

**Patentansprüche für folgende Vertragsstaaten : BE, CH, DE, DK, FR, GB, IT, LI, NL, SE**

1. Hydrophile, quellbare Pfropfpolymerisate, die in statistischer Verteilung zu 0,5 bis 20 Gew.% aus Resten der allgemeinen Formel I

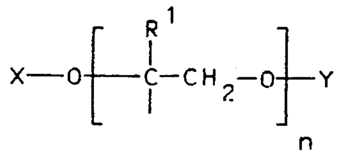

(I)

zu 79 bis 99 Gew.% aus eine saure Gruppe enthaltenden Resten der allgemeinen Formel II

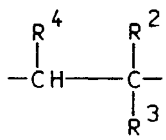

(II)

und zu 0,1 bis 2 Gew.% aus Resten eines Vernetzers, die aus Monomeren mit mindestens zwei olefinisch ungesättigten Doppelbindungen hervorgegangen sind, bestehen, wobei
X ($C_1$-$C_{22}$)-Alkyl, Aryl, Aralkyl oder Y,
Y $COCH_3$, $CH_2COOR^2$, $COCH_2CH_2COOH$,

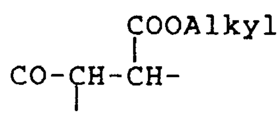

$COOR^2$, $CH_2COCH_2COOR^2$,

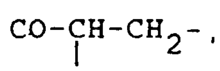

$SO_3H$ oder

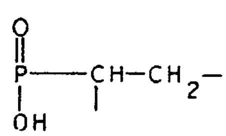

,

n 2 bis 300,
$R^1$ Wasserstoff oder Methyl,
$R^2$ unabhängig voneinander Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

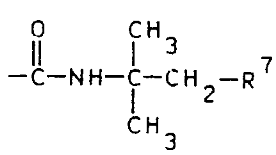

worin $R^7$ für die Sulfonylgruppe oder die Phosphonylgruppe steht und $R^4$ Wasserstoff, Methyl, Ethyl oder die Carboxylgruppe bedeuten.

**2.** Pfropfpolymerisate gemäß Anspruch 1, dadurch gekennzeichnet, daß sie in statistischer Verteilung zu 1 bis 10,5 Gew.% aus Resten der allgemeinen Formel I, zu 88 bis 98,5 Gew.% aus Resten der allgemeinen Formel II und zu 0,3 bis 1,5 Gew.% aus vernetzenden Strukturen bestehen.

**3.** Pfropfpolymerisate gemäß Ansprüchen 1 und/oder 2, dadurch gekennzeichnet, daß sich die Reste der allgemeinen Formel I hinsichtlich des Restes $R^1$ und/oder der Zahl n voneinander unterscheiden.

**4.** Pfropfpolymerisate nach einem oder mehreren der Ansprüche 1 bis 3, dadurch gekennzeichnet, daß in den Resten der allgemeinen Formel II $R^2$ Wasserstoff oder Methyl, $R^3$ die Carboxylgruppe, die Sulfonylgruppe oder die Phosphonylgruppe und $R^4$ Wasserstoff bedeuten.

**5.** Pfropfpolymerisate nach einem oder mehreren der Ansprüche 1 bis 4, dadurch gekennzeichnet, daß in den Resten der allgemeinen Formel II $R^3$ die Carboxylgruppe bedeutet.

**6.** Pfropfpolymerisate nach einem oder mehreren der Ansprüche 1 bis 5, dadurch gekennzeichnet, daß sich die vernetzenden Strukturen von Monomeren mit mindestens zwei Alkenylgruppen oder mindestens zwei Alkenoylgruppen, insbesondere van Trimethylolpropantriacrylat, Tetraallyloxyethan oder Methylenbisacrylamid ableiten.

**7.** Verfahren zur Herstellung der in den Ansprüchen 1 bis 6 beanspruchten Pfropfpolymerisate, dadurch gekennzeichnet, daß 0,5 bis 20 Gew.%, vorzugsweise 0,5 bis 15, insbesondere 1 bis 10,5 Gew.%, einer Polyalkylenoxidverbindung der allgemeinen Formel I a

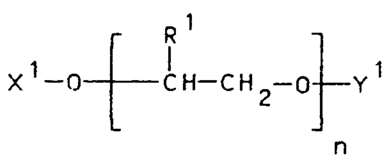

(Ia)

oder gegebenenfalls ein Alkali-, Ammonium- oder Aminsalz davon, 79 bis 99 Gew.%, vorzugsweise 84 bis 99, insbesondere 88 bis 98,5 Gew.%, einer ungesättigten Säure der allgemeinen Formel II a

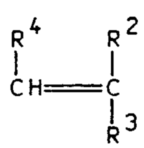

(IIa)

oder ein Alkali-, Ammonium- oder Aminsalz davon, und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen wobei
$X^1$ ($C_1$-$C_{22}$)-Alkyl, Aryl, Aralkyl oder Y,
$Y^1$ $COCH_3$, $CH_2COOR^2$, $COCH_2CH_2COOH$, CO-CH = CH-COOAlkyl, $COOR^2$, $CH_2COCH_2COOR^2$, CO-CH = $CH_2$, $SO_3H$ oder

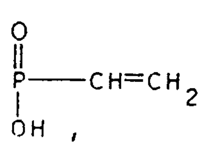

,

und $R^1$ bis $R^4$ und n wie im Anspruch 1 definiert sind, unter den Bedingungen der Gelpolymerisation umgesetzt werden.

**8.** Verwendung der in den Ansprüchen 1 bis 7 beanspruchten Pfropfpolymerisate als Absorptionsmittel für Wasser und wäßrige Lösungen.

**9.** Verwendung der in den Ansprüchen 1 bis 7 beanspruchten Pfropfpolymerisate gemäß Anspruch 8, dadurch gekennzeichnet, daß die Pfropfpolymerisate in Hygieneartikeln wie Windeln, Tampons oder Damenbinden eingesetzt werden.

**Patentansprüche für folgenden Vertragsstaat : ES**

**1.** Verfahren zur Herstellung hydrophiler, quellbarer Pfropfpolymerisate, die in statistischer Verteilung zu 0,5 bis 20 Gew.% aus Resten der allgemeinen Formel I

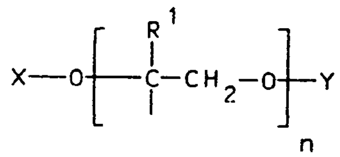

(I)

zu 79 bis 99 Gew.% aus eine saure Gruppe enthaltenden Resten der allgemeinen Formel II

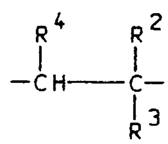

(II)

und zu 0,1 bis 2 Gew.% aus Resten eines Vernetzers, die aus Monomeren mit mindestens zwei olefinisch ungesättigten Doppelbindungen hervorgegangen sind, bestehen, wobei
X $(C_1\text{-}C_{22})$-Alkyl, Aryl, Aralkyl oder Y,
Y $COCH_3$, $CH_2COOR^2$, $COCH_2CH_2COOH$,

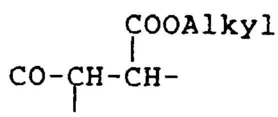

$COOR^2$, $CH_2COCH_2COOR^2$,

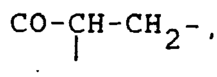

$SO_3H$ oder

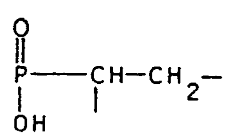

,

n 2 bis 300,
$R^1$ Wasserstoff oder Methyl,
$R^2$ unabhängig voneinander Wasserstoff, Methyl oder - Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

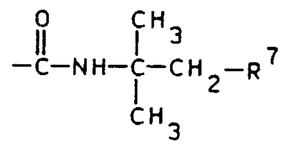

worin $R^7$ für die Sulfonylgruppe oder die Phosphonylgruppe steht und $R^4$ Wasserstoff, Methyl, Ethyl oder die Carboxylgruppe bedeuten, dadurch gekennzeichnet, daß 0,5 bis 20 Gew.%, vorzugsweise 0,5 bis 15, insbesondere 1 bis 10,5 Gew.%, einer Polyalkylenoxidverbindung der allgemeinen Formel Ia

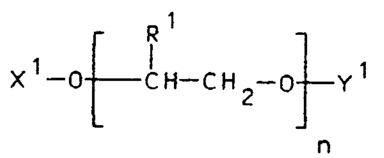 (Ia)

oder gegebenenfalls ein Alkali-, Ammonium- oder Aminsalz davon,
79 bis 99 Gew.%, vorzugsweise 84 bis 99, insbesondere 88 bis 98,5 Gew.%, einer ungesättigten Säure der allgemeinen Formel IIa

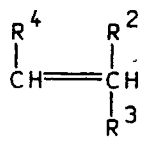 (IIa)

oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei
$X^1$ ($C_1$-$C_{22}$)-Alkyl, Aryl, Aralkyl oder Y,
$Y^1$ $COCH_3$, $CH_2COOR^2$, $COCH_2CH_2COOH$, CO-CH = CH-COOAlkyl, $COOR^2$, $CH_2COCH_2COOR^2$, CO-CH = $CH_2$, $SO_3H$ oder

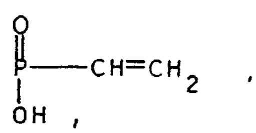

und $R^1$ bis $R^4$ und n wie oben definiert sind, unter den Bedingungen der Gelpolymerisation umgesetzt werden.

2. Verfahren gemäß Anspruch 1, dadurch gekennzeichnet, daß die Pfropfpolymerisate in statistischer Verteilung zu 1 bis 10,5 Gew.% aus Resten der allgemeinen Formel I, zu 88 bis 98,5 Gew.% aus Resten der allgemeinen Formel II und zu 0,3 bis 1,5 Gew.% aus vernetzenden Strukturen bestehen.

3. Verfahren gemäß Ansprüchen 1 und/oder 2, da-durch gekennzeichnet, daß sich die Reste der allgemeinen Formel I hinsichtlich des Restes $R^1$ und/oder der Zahl n voneinander unterscheiden.

4. Verfahren nach einem oder mehreren der Ansprüche 1 bis 3, dadurch gekennzeichnet, daß in den Resten der allgemeinen Formel II $R^2$ Wasserstoff oder Methyl, $R^3$ die Carboxylgruppe, die Sulfonylgruppe oder die Phosphonylgruppe und $R^4$ Wasserstoff bedeuten.

5. Verfahren nach einem oder mehreren der Ansprüche 1 bis 4, dadurch gekennzeichnet, daß in den Resten der allgemeinen Formel II $R^3$ die Carboxylgruppe bedeutet.

6. Verfahren nach einem oder mehreren der Ansprüche 1 bis 5, dadurch gekennzeichnet, daß sich die vernetzenden Strukturen von Monomeren mit mindestens zwei Alkenylgruppen oder mindestens zwei Alkenoylgruppen, insbesondere von Trimethylolpropantriacrylat, Tetraallyloxyethan oder Methylenbisacrylamid ableiten.

7. Verwendung der nach einem der Verfahren der Ansprüche 1 bis 6 hergestellten Pfropfpolymerisate als Absorptionsmittel für Wasser und wäßrige Lösungen.

8. Verwendung der nach einem der Verfahren der Ansprüche 1 bis 7 hergestellten Pfropfpolymerisate gemäß Anspruch 7, dadurch gekennzeichnet, daß die Pfropfpolymerisate in Hygieneartikeln wie Windeln, Tampons oder Damenbinden eingesetzt werden.

**Claims**

**Claims for the following Contracting States : BE, CH, DE, DK, FR, GB, IT, LI, NL, SE**

1. Hydrophilic, swellable graft copolymers composed, in random distribution, of 0.5 to 20 % by weight of radicals of the general formula I

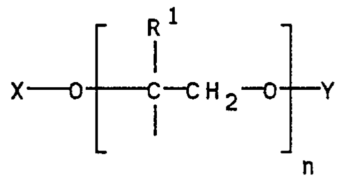

(I)

79 to 99 % by weight of radicals containing an acidic group, of the general formula II

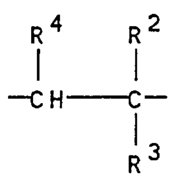

(II)

and 0.1 to 2 % by weight of radicals of a crosslinking agent which are derived from monomers having at least two olefinically unsaturated double bonds, where
X denotes $(C_1\text{-}C_{22})$-alkyl, aryl, aralkyl oder Y,
Y denotes $COCH_3$, $CH_2COOR^2$, $COCH_2CH_2COOH$,

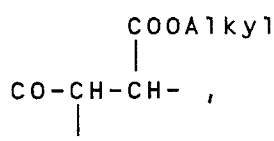

$COOR^2$, $CH_2COCH_2COOR^2$,

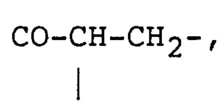

$SO_3H$ or

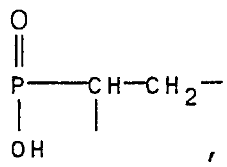,

n denotes 2 to 300,
$R^1$ denotes hydrogen or methyl,
$R^2$ independently of one another denotes hydrogen, methyl or ethyl,
$R^3$ denotes the carboxyl group, the sulphonyl group, the phosphonyl group, which optionally may be esterified with alkanol having 1 to 4 carbon atoms, or denotes a group of the formula

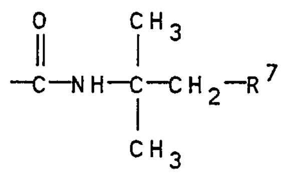

in which $R^7$ represents the sulphonyl group or the phosphonyl group, and $R^4$ denotes hydrogen, methyl, ethyl or the carboxyl group.

2. Graft copolymers according to Claim 1, characterized in that they are composed, in random distribution, of 1 to 10,5 % by weight of radicals of the general formula I, 88 to 98.5 % by weight of radicals of the general formula II and 0.3 to 1.5 % by weight of crosslinking structures.

3. Graft copolymers according to Claims 1 and/or 2, characterized in that the radicals of the general formula I differ with regard to the radical $R^1$ and/or the number n.

4. Graft copolymers according to one or more of Claims 1 to 3, characterized in that $R^2$ in the radicals of the general formula II denotes hydrogen or methyl, $R^3$ denotes the carboxyl group, the sulphonyl group or the phosphonyl group and $R^4$ denotes hydrogen.

5. Graft copolymers according to one or more of Claims 1 to 4, characterized in that $R^3$ in the radicals of the general formula II denotes the carboxyl group.

6. Graft copolymers according to one or more of Claims 1 to 5, characterized in that the crosslinking structures are derived from monomers having at least two alkenyl groups or at least two alkenoyl groups, in particular from trimethylolpropane triacrylate, tetraallyloxyethane or methylenebisacrylamide.

7. Process for the preparation of the graft copolymers claimed in Claims 1 to 6, characterized in that 0.5 to 20 % by weight, preferably 0.5 to 15, in particular 1 to 10.5, % by weight of a polyalkylene oxide compound of the general formula Ia

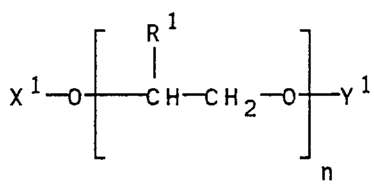 (Ia)

or optionally an alkali metal salt, ammonium salt or amine salt thereof, 79 to 99 % by weight, preferably 84 to 99, in particular 88 to 98.5, % by weight of an unsaturated acid of the general formula IIa

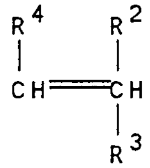

(IIa)

or an alkali metal salt, ammonium salt or amine salt thereof, and 0.1 to 2 % by weight, preferably 0.1 to 1.8, in particular 0.3 to 1.5, % by weight of a monomer having at least two olefinically unsaturated double bonds, where
$X^1$ denotes ($C_1$-$C_{22}$)-alkyl, aryl, aralkyl or Y,
$Y^1$ denotes $COCH_3$, $CH_2COOR^2$, $COCH_2CH_2COOH$, CO-CH = CH-COOalkyl, $COOR^2$, $CH_2COCH_2COOR^2$, CO-CH = $CH_2$, $SO_3H$ or

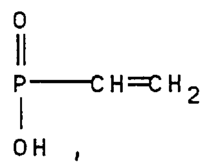

,

and $R^1$ to $R^4$ and n are as defined in Claim 1, are reacted under the conditions of gel polymerization.

8. The use of the graft copolymers claimed in Claims 1 to 7 as absorbents for water and aqueous solutions.

9. The use ofthe graft copolymers claimed in Claims 1 to 7 according to Claim 8, characterized in that the graft copolymers are used in sanitary items such as diapers, tampons or sanitary towels.

**Claims for the following Contracting State : ES**

1. Process for preparing hydrophilic, swellable graft copolymers composed, in random distribution, of 0.5 to 20 % by weight of radicals of the general formula I

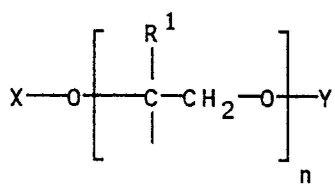

(I)

79 to 99 % by weight of radicals containing an acidic group, of the general formula II

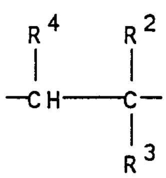

(II)

and 0.1 to 2 % by weight of radicals of a crosslinking agent which are derived from monomers having at least two olefinically unsaturated double bonds, where
X denotes ($C_1$-$C_{22}$)-alkyl, aryl, aralkyl oder Y,
Y denotes $COCH_3$, $CH_2COOR^2$, $COCH_2CH_2COOH$,

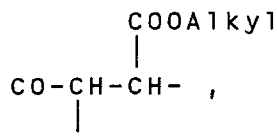

$COOR^2$, $CH_2COCH_2COOR^2$,

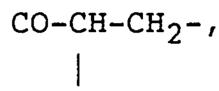

$SO_3H$ or

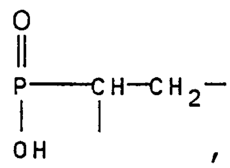

n denotes 2 to 300,
$R^1$ denotes hydrogen or methyl,
$R^2$ independently of one another denotes hydrogen, methyl or ethyl,
$R^3$ denotes the carboxyl group, the sulphonyl group, the phosphonyl group, which optionally may be esterified with alkanol having 1 to 4 carbon atoms, or denotes a group of the formula

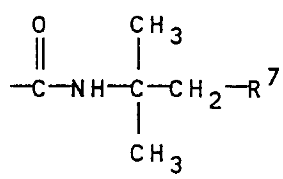

in which $R^7$ represents the sulphonyl group or the phosphonyl group, and $R^4$ denotes hydrogen, methyl, ethyl or the carboxyl group, characterized in that 0.5 to 20 % by weight, preferably 0.5 to 15, in particular 1 to 10.5, % by weight of a polyalkylene oxide compound of the general formula Ia

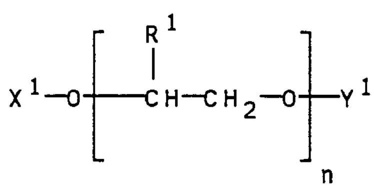

(Ia)

or optionally an alkali metal salt, ammonium salt or amine salt thereof, 79 to 99 % by weight, preferably 84 to 99, in particular 88 to 98.5, % by weight of an unsaturated acid of the general formula IIa

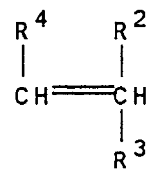

(IIa)

or an alkali metal salt, ammonium salt or amine salt thereof, and 0.1 to 2 % by weight, preferably 0.1 to 1.8, in particular 0.3 to 1.5, % by weight of a monomer having at least two olefinically unsaturated double bonds, where

$X^1$ denotes ($C_1$-$C_{22}$)-alkyl, aryl, aralkyl or Y,

$Y^1$ denotes $COCH_3$, $CH_2COOR^2$, $COCH_2CH_2COOH$, CO-CH = CH-COOalkyl, $COOR^2$, $CH_2COCH_2COOR^2$, CO-CH = $CH_2$, $SO_3H$ or

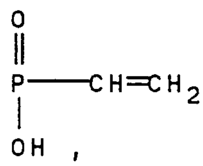

and $R^1$ to $R^4$ and n are as defined in Claim 1, are reacted under the conditions of gel polymerization.

2. Process according to Claim 1, characterized in that the graft polymers are composed, in random distribution, of 1 to 10,5 % by weight of radicals of the general formula I, 88 to 98.5 % by weight of radicals of the general formula II and 0.3 to 1.5 % by weight of crosslinking structures.

3. Process according to Claims 1 and/or 2, characterized in that the radicals of the general formula I differ with regard to the radical $R^1$ and/or the number n.

4. Process according to one or more of Claims 1 to 3, characterized in that $R^2$ in the radicals of the general formula II denotes hydrogen or methyl, $R^3$ denotes the carboxyl group, the sulphonyl group or the phosphonyl group and $R^4$ denotes hydrogen.

5. Process according to one or more of Claims 1 to 4, characterized in that $R^3$ in the radicals of the general formula II denotes the carboxyl group.

6. Process according to one or more of Claims 1 to 5, characterized in that the crosslinking structures are derived from monomers having at least two alkenyl groups or at least two alkenoyl groups, in particular from trimethylolpropane triacrylate, tetraallyloxyethane or methylenebisacrylamide.

7. The use of the graft copolymers prepared according to a process of Claims 1 to 6 as absorbents for water and aqueous solutions.

8. The use of the graft copolymers according to Claim 7 prepared according to a process of Claims 1 to 7, characterized in that the graft copolymers are used in sanitary items such as diapers, tampons or sanitary towels.

**Revendications**

**Revendications pour les Etats contractants suivants : BE, CH, DE, DK, FR, GB, IT, LI, NL, SE**

1. Polymères greffés gonflables hydrophiles, qui se composent d'une répartition statistique de 0,5 à 20 % en poids de radicaux de formule générale I :

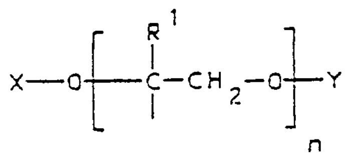

(I)

de 79 à 99 % en poids de radicaux de formule générale II contenant un groupe acide :

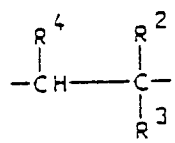

(II)

et de 0,1 à 2 % en poids de radicaux d'un agent réticulant, qui proviennent de monomères avec au moins deux doubles liaisons à insaturation éthylénique, où X est les alkyle en $C_1$-$C_{22}$, aryle, aralkyle ou Y,
Y représente
$COCH_3$, $CH_2COOR^2$, $COCH_2CH_2COOH$,

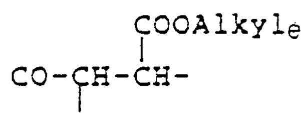

$COOR^2$, $CH_2COCH_2COOR^2$,

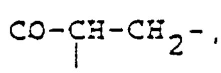

$SO_3H$ ou

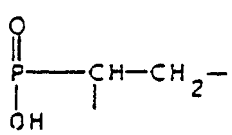

,

n va de 2 à 300,
$R^1$ est l'hydrogène ou un méthyle,
$R^2$ indépendamment les uns des autres représente l'hydrogène, le méthyle ou l'éthyle,
$R^3$ représente les groupes carboxyle, sulfonyle, phosphonyle, qui peuvent éventuellement être estérifiés par un alcanol comportant de 1 à 4 atomes de carbone, ou un groupe de formule :

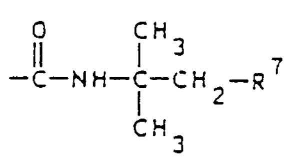

dans laquelle $R^7$ représente le groupe sulfonyle ou le groupe phosphonyle, et
$R^4$ signifie les hydrogène, méthyle, éthyle ou le groupe carboxyle.

2. Polymères greffés selon la revendication 1, caractérisés en ce qu'ils se composent d'une répartition statistique de 1 à 10,5 % en poids de radicaux de formule générale I, de 88 à 98,5 % en poids de radicaux de formule générale II et de 0,3 à 1,5 % en poids de structures réticulantes.

3. Polymères greffés selon la revendication 1 et/ou 2, caractérisés en ce que les radicaux de formule générale I diffèrent l'un de l'autre en ce qui concerne les radicaux $R^1$ et/ou le nombre n.

4. Polymères greffés selon une ou plusieurs des revendications 1 à 3, caractérisés en ce que dans les radicaux de formule générale II, $R^2$ signifie l'hydrogène ou le méthyle, $R^3$ les groupes carboxyle, sulfonyle ou phosphonyle, et $R^4$ l'hydrogène.

5. Polymères greffés selon une ou plusieurs des revendications 1 à 4, caractérisés en ce que dans les radicaux de formule générale II, $R^3$ signifie le groupe carboxyle.

6. Polymères greffés selon une ou plusieurs des revendications 1 à 5, caractérisés en ce que les structures réticulantes dérivent de monomères comportant au moins deux groupes alcényle ou au moins deux groupes alcénoyle, plus particulièrement du triacrylate de triméthylolpropane, du tétra-allyloxy-éthane ou du méthylène-bis-acrylamide.

7. Procédé pour la préparation des polymères greffés revendiqués dans les revendications 1 à 6, caractérisé en ce qu'on fait réagir de 0,5 à 20 % en poids, de préférence de 0,5 à 15, plus particulièrement de 1 à 10,5 % en poids d'un composé d'oxyde de polyalkylène de formule générale Ia :

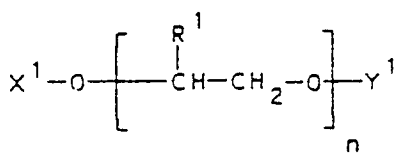

(Ia)

ou éventuellement d'un sel alcalin, d'un sel d'ammonium ou d'un sel d'amine de celui-ci, de 79 à 99 % en poids, de préférence de 84 à 99, plus particulièrement de 88 à 98,5 % en poids d'un acide insaturé de formule générale IIa :

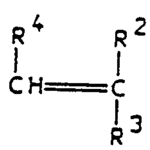

(IIa)

ou d'un de ses sels alcalins, d'ammonium ou d'amine et de 0,1 à 2 %, de préférence de 0,1 à 1,8, plus particulièrement de 0,3 à 1,5 % en poids, d'un monomère avec au moins deux doubles liaisons à insaturation oléfinique, où

$X^1$ représente un alkyle en $C_1$-$C_{22}$, un aryle, un aralkyle ou Y,

$Y^1$ représente

$COCH_3$, $CH_2COOR^2$, $COCH_2CH_2COOH$, CO-CH = CH-COOAlkyle, $COOR^2$, $CH_2COCH_2COOR^2$, CO-CH = $CH_2$, $SO_3H$ ou

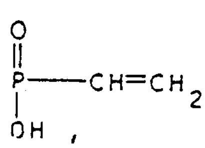

et $R^1$ à $R^4$ et le nombre n ont les significations définies dans la revendication 1, dans des conditions de polymérisation en gel.

8. Utilisation des polymères greffés revendiqués dans les revendications 1 à 7, en tant que produit absorbant l'eau et des solutions aqueuses.

9. Utilisation des polymères greffés revendiqués dans les revendications 1 à 7, selon la revendication 8, caractérisée en ce que les polymères greffés sont utilisés dans des articles hygiéniques comme des couches, tampons ou serviettes périodiques.

**Revendications pour l'Etat contractant suivant : ES**

1. Procédé pour la préparation de polymères greffés gonflables hydrophiles, qui se composent d'une répartition statistique de 0,5 à 20 % en poids de radicaux de formule générale I :

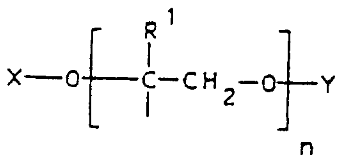

(I)

de 79 à 99 % en poids de radicaux de formule générale II contenant un groupe acide :

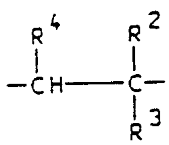

(II)

et de 0,1 à 2 % en poids de radicaux d'un agent réticulant, qui proviennent de monomères avec au moins deux doubles liaisons à insaturation éthylénique, où
X est les alkyle en $C_1$-$C_{22}$, aryle, aralkyle ou Y,
Y représente
$COCH_3$, $CH_2COOR^2$, $COCH_2CH_2COOH$,

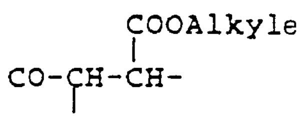

$COOR^2$, $CH_2COCH_2COOR^2$,

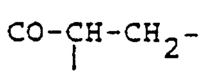,

$SO_3H$ ou

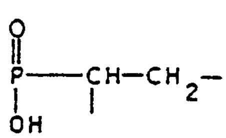,

n va de 2 à 300,
$R^1$ est l'hydrogène ou un méthyle,
$R^2$ indépendamment les uns des autres représente l'hydrogène, le méthyle ou l'éthyle,
$R^3$ représente les groupes carboxyle, sulfonyle, phosphonyle, qui peuvent éventuellement être estérifiés

par un alcanol comportant de 1 à 4 atomes de carbone, ou un groupe de formule :

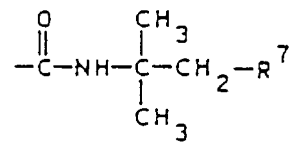

dans laquelle $R^7$ représente le groupe sulfonyle ou le groupe phosphonyle, et
$R^4$ signifie les hydrogène, méthyle, éthyle ou le groupe carboxyle, caractérisé en ce qu'on fait réagir de 0,5 à 20 % en poids, de préférence de 0,5 à 15, plus particulièrement de 1 à 10,5 % en poids d'un composé d'oxyde de polyalkylène de formule générale Ia :

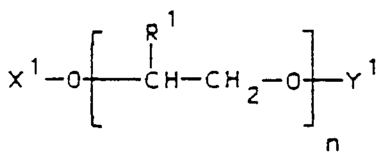

(Ia)

ou éventuellement d'un sel alcalin, d'un sel d'ammonium ou d'un sel d'amine de celui-ci,
de 79 à 99 % en poids, de préférence de 84 à 99, plus particulièrement de 88 à 98,5 % en poids, d'un acide insaturé de formule générale IIa :

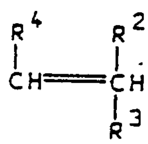

(IIa)

ou d'un de ses sels alcalins, d'ammonium ou d'amine, et de 0,1 à 2 %, de préférence de 0,1 à 1,8, plus particulièrement de 0,3 à 1,5 % en poids, d'un monomère avec au moins deux doubles liaisons à insaturation oléfinique, où
$X^1$ représente les alkyle en $C_1$-$C_{22}$, aryle, aralkyle ou Y,
$Y^1$ représente
$COCH_3$, $CH_2COOR^2$, $COCH_2CH_2COOH$, CO-CH = CH-COOAlkyle, $COOR^2$, $CH_2COCH_2COOR^2$, CO-CH = $CH_2$, $SO_3H$ ou

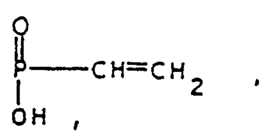

et $R^1$ à $R^4$ et le nombre n sont définis ci-dessus, dans des conditions de polymérisation en gel.

**2.** Procédé selon la revendication 1, caractérisé en ce que les polymères greffés se composent d'une répartition statistique de 1 à 10,5 % en poids de radicaux de formule générale I, de 88 à 98,5 % en poids de radicaux de formule générale II, et de 0,3 à 1,5 % en poids de structures réticulantes.

**3.** Procédé selon les revendications 1 et/ou 2, caractérisé en ce que les radicaux de formule générale I diffèrent l'un de l'autre en ce qui concerne les radicaux $R^1$ et/ou le nombre n.

**4.** Procédé selon une ou plusieurs des revendications 1 à 3, caractérisé en ce que dans les radicaux de formule générale II, $R^2$ signifie l'hydrogène ou le méthyle, $R^3$ les groupes carboxyle, sulfonyle ou phosphonyle, et $R^4$ l'hydrogène.

**5.** Procédé selon une ou plusieurs des revendications 1 à 4, caractérisé en ce que dans les radicaux de formule générale II, $R^3$ signifie le groupe carboxyle.

**6.** Procédé selon une ou plusieurs des revendications 1 à 5, caractérisé en ce que les structures réticulantes dérivent de monomères comportant au moins deux groupes alcényle ou au moins deux groupes alcénoyle, plus particulièrement du triacrylate de triméthylol-propane, du tétra-allyloxy-éthane ou du méthylène-bis-acrylamide.

**7.** Utilisation des polymères greffés préparés selon un procédé des revendications 1 à 6, en tant que produit absorbant l'eau et des solutions aqueuses.

**8.** Utilisation des polymères greffés préparés selon un des procédés des revendications 1 à 7, selon la revendication 7, caractérisée en ce que les polymères greffés sont utilisés dans des articles hygiéniques comme des couches, tampons ou serviettes périodiques.